# FASCICULE DE BREVET EUROPEEN

(11) **EP 2 101 730 B1**
(45) Date de publication et mention de la délivrance du brevet: **21.07.2010**
(21) Numéro de dépôt: 07871968.9
(22) Date de dépôt: 19.12.2007
(51) Int. Cl.: A61K 9/12, A61K 31/167, A61P 29/02, A61P 25/06, A61K 9/08

(54) **FORME GALENIQUE POUR L'ADMINISTRATION PAR VOIE TRANS-MUQUEUSE DE PARACETAMOL**
GALENISCHE FORM ZUR TRANSMUKOSALEN VERABREICHUNG VON PARACETAMOL
GALENIC FORM FOR THE TRANSMUCOSAL DELIVERY OF PARACETAMOL

(30) Priorité: 21.12.2006 FR 0655773
(43) Date de publication de la demande: 23.09.2009
(73) Titulaire: Perovitch, Philippe, 33680 Le Temple (FR); Maury, Marc, 33160 Saint Medard en Jalles (FR)
(72) Inventeur: Perovitch, Philippe, 33680 Le Temple (FR); Maury, Marc, 33160 Saint Medard en Jalles (FR)
(74) Mandataire: Cenatiempo, Julie Adeline Anne
(86) Numéro de dépôt international: PCT/FR2007/052555
(87) Numéro de publication internationale: WO 2008/087323

(56) Documents cités:
- WO-A-00/41692
- WO-A-95/23591
- US-A- 5 981 591
- US-B1- 6 767 925

## Description

La présente invention concerne une forme galénique pour l'administration systémique instantanée par voie trans-muqueuse de médicaments antalgiques-antipyrétiques principalement à base de Paracétamol.

L'invention se rapporte également à un procédé de fabrication et aux utilisations de la forme galénique.

Le Paracétamol est une très petite molécule de caractère lipophile, qui possède une activité pharmacologique exclusivement antalgique et antipyrétique, essentiellement centrale, au niveau des voies et récepteurs cérébraux régulant la douleur et la fièvre. Très couramment utilisé pour traiter les états fiévreux et les douleurs d'intensité moyenne de type céphalées, dysménorrhées, etc., il est généralement administré, seul ou en association avec d'autres principes actifs thérapeutiques, en dosages unitaires de 500mg ou 1g par voie orale.

Or une telle d'administration n'est pas satisfaisante.

En effet, lorsque les molécules de Paracétamol sont introduites dans le tube digestif et l'estomac, elles subissent l'effet dit de premier passage digestif, altérations et déperditions liées au milieu stomacal ou aux variations des physiologies intestinales. Elles sont ensuite soumises à un effet dit de « premier passage hépatique » qui provoque leur métabolisation et/ou leur dégradation plus ou moins intense, avec constitution de nombreux métabolites, pour la plupart inactifs ou toxiques, sans compter que le Paracétamol est toxique pour le foie lorsqu'il est administré à un dosage supérieur à 4g.

La dose de principe actif véritablement biodisponible est donc faible, évoluée en moyenne entre 60 et 75% de la dose administrée, sans présumer de nombreuses variabilités inter et intra-individuelles.

En outre, le temps de demi-vie du Paracétamol est relativement bref, situé entre 1 et 3 heures. Très peu liée aux protéines plasmatiques, du fait de sa nature lipophile la molécule de Paracétamol est très largement dans la plupart des compartiments de l'organisme, Ainsi, une part importante de la dose administrée va se trouver diluée dans le réseau vasculaire de l'organisme en même temps que dispersée dans les espaces extra-vasculaires organiques et surtout tissulaires, réduisant la part réellement disponible de Paracétamol susceptible d'accéder aux récepteurs centraux cérébraux pour y exercer son activité pharmacologique, de courte durée d'action.

En outre, le début de l'efficacité thérapeutique pour le patient intervient entre 30 et 60 minutes après la prise, correspondant au délai d'absorption digestive, de métabolisation et de diffusion vasculaire, puis tissulaire.

De fait, il apparaît deux problèmes majeurs.

Le premier problème est qu'il faut administrer une dose suffisante au patient tenant compte de la dilution et de la dispersion dans l'organisme, pour que la partie significativement active qui atteint les effecteurs centraux soit efficace. Le second est le temps de latence dû à la métabolisation et à la diffusion dans l'organisme avant que la molécule agisse et que le patient en ressente les bienfaits.

Des solutions ont tenté d'être apportées, mais aucune n'est satisfaisante.

La demande WO 00/41692 par exemple décrit du Paracétamol formulé dans une solution. Toutefois, cette solution est avalée par le patient. Le Paracétamol subit donc toujours les problématiques du passage digestif et les inconvénients mentionnés, ci-avant.

On connaît également les demandes US-5.981.591 et US-6.767.925 qui proposent des formulations sous forme de spray destinées à une administration per-muqueuse buccale. Ces formulations ne permettent que l'administration d'une dose infime de Paracétamol par pulvérisation qui est en outre en grande partie avalée, l'administration par spray entraînant la déperdition/déglutition après mélange salivaire réflexe.

Il subsiste donc un besoin pour une formulation galénique permettant d'administrer une quantité immédiatement biodisponible de Paracétamol, seul ou en association avec d'autres principes actifs, de façon à pouvoir exercer très rapidement et efficacement une activité thérapeutique notamment antalgique et anti-pyrétique.

C'est ce à quoi répond la présente invention en proposant une forme galénique pour l'administration par voie trans-muqueuse d'au moins un principe actif, caractérisée en ce que ledit principe actif est du Paracétamol en état de dissolution stable et complète dans une solution hydroalcoolique telle que décrite dans la revendication 1, de façon à permettre une absorption rapide dudit principe actif à travers les muqueuses de la cavité buccale et/ou de l'oropharynx. L'invention est maintenant décrite en détails.

Par voie trans-muqueuse, on entend tout franchissement passif à travers les muqueuses linguales, sublinguales, gingivales, palatines, jugales, ou toutes autres muqueuses constitutives de la cavité buccale et de l'oropharynx.

Par état de dissolution stable et complète, on entend un état de dissolution restituant le principe actif à l'état moléculaire et faiblement ionisé dans son milieu de dissolution, état de dissolution prévenant toute éventualité d'une recristallisation inopportune.

De façon préférentielle, la forme galénique selon l'invention se présente sous forme d'une solution hydroalcoolique comprenant entre 10 et 70% d'éthanol en masse et une teneur en eau comprise entre 30 et 90%. Le passage dans la circulation systémique centrale des molécules de Paracétamol formulées selon l'invention, s'effectue donc en solution hydroalcoolique de degré variable d'alcool, préférentiellement compris entre 10° et 70°, encore plus préférentiellement entre 25° et 55°.

Selon une caractéristique majeure de l'invention, l'alcool, présent à au moins 10% en masse, ne joue pas seulement le rôle de solvant, mais également celui de promoteur d'une absorption per-muqueuse accélérée, dont la vitesse croit en fonction de l'élévation du degré d'alcool utilisé.

Selon un mode de réalisation préféré de l'invention, la solution hydroalcoolique est réalisée à base d'eau et d'éthanol.

Le coefficient de dissolution du Paracétamol dans l'éthanol permet d'obtenir une dissolution complète dudit principe actif à hauteur de 250mg de Paracétamol pour 2 ml d'éthanol à 45%. Ce coefficient peut être modulé en fonction du degré d'alcool et du ratio eau/éthanol utilisé. A titre d'exemple un mélange eau/éthanol à 45% à 50/50 en volume fait apparaître une meilleure solubilisation du Paracétamol, comparée à celle obtenue dans l'éthanol pur.

Selon un autre mode de réalisation, la solution hydroalcoolique peut être réalisée à base d'eau et d'alcool isopropylique.

La solution hydroalcoolique selon l'invention peut comprendre également un ou plusieurs adjuvants de dissolution du ou des principes actifs, comme un polymère de type PEG (Poly-éthylène Glycol) de faible poids moléculaire, l'alcool isopropylique, un tensioactif tel que le Crémophor ou un Polysorbate, et/ou des mélanges alcool-huile. Elle peut également contenir un arôme ou un édulcorant pour adoucir la sensation gustative.

Selon un mode de réalisation particulier, la forme galénique selon l'invention peut aussi comprendre un agent correcteur de pH.

Préférentiellement, l'agent correcteur de pH est choisi parmi les carbonates et bicarbonates de sodium, les phosphates monosodique ou disodique, la triéthanolamine, la soude (NaOH) et la potasse (KOH).

La forme galénique selon l'invention permet aux molécules de Paracétamol et aux éventuels autres principes actifs de franchir passivement les muqueuses de l'oropharynx dans un délai inférieur à 10 secondes après administration.

Ce délai d'absorption très rapide permet de prévenir toute stagnation de la solution et du Paracétamol dans l'atmosphère buccale ainsi que son mélange inopportun avec de la salive susceptible de l'altérer, ce qui introduirait une rupture dans la continuité et la stabilité de la dissolution du principe actif. Ce court délai permet également de prévenir toute déglutition réflexe de la solution et du Paracétamol qu'elle contient.

Le passage trans-muqueux du Paracétamol présenté en état de dissolution selon l'invention du côté de la membrane épithéliale externe, constituée de structures phospho-lipidiques qui absorbent passivement les molécules lipophiles par affinité élective, est basé sur un appel osmotique vers l'autre côté de ladite membrane, auquel participent ensemble la concentration en principe actif dissous et celle de la solution alcoolique considérée. L'appel osmotique est d'autant plus vivace et puissant que le degré d'alcool qui sert de promoteur d'absorption est élevé. Dans le cas particulier du Paracétamol, selon l'invention, un degré d'alcool adapté est compris entre 10° et 70°, préférentiellement entre 25° et 55°. Ceci permet d'assurer simultanément l'obtention et le réglage du meilleur coefficient de dissolution et de stabilisation du Paracétamol ainsi que la promotion de son passage per-muqueux dans un délai d'une dizaine de secondes. Un mode de réalisation particulièrement adapté correspond à 1ml de solution hydro alcoolique à un degré d'alcool de 45° pour 125mg de Paracétamol.

Les muqueuses de la bouche et de l'oropharynx possèdent un réseau de micro-vaisseaux très dense, quasi-spongieux, si bien que les molécules, tant de solvant alcoolique que de Paracétamol dissout, qui franchissent la membrane épithéliale, sont instantanément capturées par la micro-circulation sanguine et emportées vers les veines sublinguales. Ce phénomène est accentué par la présence de l'alcool qui provoque une vasodilatation et un accroissement du débit micro-vasculaire des muqueuses.

Il n'y a donc jamais d'équilibre de part et d'autre de la membrane : la concentration dans la bouche reste toujours plus importante, jusqu'à épuisement du mécanisme par défaut de molécules à absorber.

Ainsi, la totalité de l'alcool et du Paracétamol et des éventuelles autres molécules qui s'y trouvent dissous selon l'invention, passent à travers la muqueuse.

L'utilisation de la forme galénique selon l'invention permet d'administrer passivement une dose de Paracétamol qui est immédiatement absorbée dès que déposée au contact de la muqueuse, pour être distribuée dans l'instant par voie vasculaire à l'ensemble de l'organisme, sans aucun délai pour son action pharmacologique et sans subir les effets préalables majeurs de passage digestif et hépatique. La forme galénique selon l'invention permet donc une immédiateté d'absorption tissulaire du Paracétamol, puis sa distribution dans la circulation centrale de l'organisme, ce qu'aucune autre forme pharmaceutique existante n'autorise, même par voie intraveineuse.

Par exemple, avec une forme galénique selon l'invention réalisée à partir de 250mg de Paracétamol solubilisé dans 2ml d'alcool éthylique à 45% M/V, on peut administrer quasi-instantanément et passivement une dose très significative de Paracétamol, 250mg, qui correspond déjà à la moitié de la dose usuellement convenue et administrée par voie orale.

La solution hydroalcoolique avec une teneur en alcool d'au moins 10% en masse selon l'invention, présente également l'avantage de protéger la formulation pharmaceutique vis-à-vis d'une contamination microbiologique sans devoir introduire d'agent(s) de conservation anti-microbien(s).

Avantageusement, la présente invention offre une grande simplicité de réalisation et une très bonne stabilité galénique : la solution eau/alcool garantit la solubilisation du Paracétamol tout en supprimant la plupart des excipients utilisés dans les formes pharmaceutiques usuelles. Elle permet donc à la fois de réduire les coûts de fabrication et de diminuer les risques d'intolérance et les possibles interactions entre principe(s) actif(s) et excipients.

De façon singulière, les délais d'action de la forme galénique selon l'invention sont très courts, en particulier comparés aux lenteurs d'absorptions des médicaments à base de Paracétamol par voie digestive. La délivrance pharmacologique quasi-instantanée permet à un patient de s'administrer lui-même un produit pour un effet presque équivalent à l'efficacité d'une injection intraveineuse en flash dans la circulation.

En outre, le Paracétamol ne rencontrant pas d'obstacle significatif pour son assimilation et sa distribution instantanée dans l'organisme, la dose de base administrée est réduite, plus proche de la dose utile pour exercer l'activité pharmacologique requise. Cette dose est bien entendu dépendante de l'effet recherché. Elle est préférentiellement comprise entre 25 et 250mg de Paracétamol, pour des volumes de solution hydroalcoolique variant de 0,5ml à 2,5ml.

Par ailleurs, la muqueuse oropharyngée disposant d'une surface totale d'absorption extrêmement large, démultipliée par son caractère de tissu villeux plissé, l'administration de la forme galénique selon l'invention est dépourvue d'un quelconque risque de déglutition intempestive ou de fausse route. En effet, elle permet un passage per-muqueux extrêmement rapide qui prévient toute dissolution salivaire ou déglutition du principe actif administré, avec l'avantage de ne pas déstabiliser les muqueuses, avec des dérivés tensioactifs par exemple, comme c'est le cas des formulations existantes.

De même, les effets de l'alcool sont insignifiants. A titre d'exemple, 2 à 4 ml d'Ethanol à 40°C ne sauraient produire qu'une alcoolémie circulante inférieure à 0,2 ou 0,4mg par litre de sang, soit 12,5 à 25 fois en dessous des tolérances légales en France de 0,5g par litre.

Un procédé de fabrication de la forme galénique selon l'invention, particulièrement adapté, comprend les étapes suivantes :
- introduire sous agitation les deux tiers du Paracétamol dans l'alcool,
- agiter la préparation, préférentiellement durant 10 à 60 minutes, pour assurer la dissolution de la première fraction de Paracétamol,
- introduire sous agitation l'eau purifiée,
- agiter la préparation, préférentiellement durant 10 à 60 minutes,
- introduire le tiers restant de Paracétamol, et
- agiter jusqu'à dissolution complète de la totalité du Paracétamol.
Préférentiellement l'alcool est initialement introduit dans une cuve en acier inoxydable munie d'un système d'agitation anti-déflagrant.

Selon un mode de réalisation, le procédé comprend également une étape de filtration de la préparation.

La forme galénique obtenue est ensuite répartie par fraction dans un conditionnement adapté.

La présente invention peut être utilisée pour l'administration systémique instantanée à doses réduites et utiles de Paracétamol seul. Une telle formulation peut être utilisée pour la réalisation d'un médicament présentant une activité thérapeutique antalgique et anti-pyrétique dans un délai très bref et à des doses très réduites par rapport aux doses traditionnelles, permettant notamment le traitement de la douleur, d'états fiévreux ou de la migraine.

La forme galénique selon l'invention peut également être utilisée pour l'administration systémique instantanée à doses réduites et utiles de Paracétamol en association avec d'autres principes actifs, par exemple destinés à traiter des affections de type grippal avec encombrements rhino-pharyngés. De telles formulations peuvent être utilisées pour la réalisation de médicaments présentant des effets spontanés apaisants et sédatifs de la douleur et de la gêne des affections grippales et rhumes.

Selon un aspect de l'invention, la forme galénique nécessite un conditionnement industriel spécifique, afin de prévenir la dégradation du ou des principes actifs au contact de l'air.

Un mode de réalisation particulier consiste à utiliser un conditionnement, préférentiellement de petite taille, plastique ou métalloplastique souple ou en verre, opaque rempli sous atmosphère d'azote, pour la protection de la stabilité de la composition et l'imperméabilité à l'oxygène et aux rayonnements. Ces conditionnements garantissent la dissolution et la stabilité dans le temps du Paracétamol et des éventuels autres principes actifs dissous en solution hydroalcoolique selon l'invention.

Pour le confort d'utilisation par le patient, pour un transport aisé, on peut préférentiellement recourir à des emballages sous forme d'étuis étanches spécifiques. Encore plus préférentiellement, la forme galénique selon l'invention est conditionnée dans des emballages unidoses de 0,5 à 30 ml, susceptibles de fournir une dose adéquate de principe actif.

De façon avantageuse, ce conditionnement est facile à transporter et permet une utilisation aisée de la forme galénique à tout moment de la journée. D'autres caractéristiques et avantages ressortiront des exemples non limitatifs qui vont suivre de l'invention.

### I. Exemples de formulation de Paracétamol seul selon l'invention

On peut citer plusieurs exemples de formulation du Paracétamol seul selon l'invention, particulièrement adaptées pour le traitement de la migraine ou d'états fiévreux.

### 1 - Exemple de formulation 0,5ml pour 25mg de Paracétamol :

- Paracétamol : 25,0mg
- eau distillée : 0,30ml
- éthanol, alcool absolu : 0,20ml
- Edulcorant qsp
- Arôme qsp

### 2 - Exemple de formulation 0,5ml pour 50mg de Paracétamol :

- Paracétamol : 50,0mg
- eau distillée : 0,30ml
- éthanol, alcool absolu : 0,20ml
- Edulcorant qsp
- Arôme qsp

### 3 - Exemple de formulation 1ml pour 100mg de Paracétamol :

- Paracétamol : 100,0mg
- eau distillée : 0,60ml
- éthanol, alcool absolu : 0,40ml
- Edulcorant qsp
- Arôme qsp

### 4 - Exemple de formulation 1ml pour 150mg de Paracétamol :

- Paracétamol : 150,0mg
- eau distillée : 0,45ml
- éthanol, alcool absolu : 0,55ml
- Edulcorant qsp - Arôme qsp

### 5 - Exemple de formulation 2ml pour 200mg de Paracétamol :

- Paracétamol : 200,0mg
- eau distillée : 1,20ml
- éthanol, alcool absolu : 0,80ml
- Edulcorant qsp
- Arôme qsp

### 6 - Exemple de formulation 2ml pour 250mg de Paracétamol :

- Paracétamol : 250,0mg
- eau distillée : 1,1ml
- éthanol, alcool absolu : 0,90ml
- Edulcorant qsp
- Arôme qsp

### II. Exemples de formulation de Paracétamol en association avec d'autres principes actifs thérapeutiques selon l'invention

La forme galénique selon l'invention peut être utilisée pour l'administration systémique instantanée à doses réduites et utiles de Paracétamol en association avec d'autres principes actifs thérapeutiques.

En particulier la forme galénique selon l'invention peut contenir en association avec du Paracétamol, toute substance lipophile compatible avec le Paracétamol en solution alcoolique, susceptible de fournir un adjuvant antalgique, décongestionnant, sédatif des voies aériennes, sinusiennes, rhino et oropharyngées.

A titre d'exemple, on peut citer des formulations selon l'invention associant à du Paracétamol :

### - de la PseudoEphédrine

La PseudoEphédrine est une petite molécule à action décongestionnante diurne. Elle peut être adjointe à une formulation à base de Paracétamol selon l'invention, à différents dosages allant par exemple de 1 à 30mg de PseudoEphédrine.

On peut citer à titre d'exemple non limitatif la formulation suivante :
- Paracétamol : 150,0mg
- PseudoEphédrine : 10,0mg
- eau distillée : 0,55ml
- éthanol, alcool absolu : 0,45ml
- Arôme qsp

### - de la Triprolidine :

La Triprolidine peut être adjointe à une formulation à base de Paracétamol selon l'invention, à différents dosages allant par exemple de 0,10 à 2,5mg de Triprolidine.

On peut citer à titre d'exemple non limitatif la formulation suivante :
- Paracétamol: 100,0mg
- Triprolidine : 0,25mg
- eau distillée : 0,60ml
- éthanol, alcool absolu : 0,40ml
- Arôme qsp

### - de la Prométhazine :

La Prométhazine peut être adjointe à une formulation à base de Paracétamol selon l'invention, à différents dosages allant par exemple de 0,15 à 25mg de Prométhazine.

### - de la Phéniramine :

La Phéniramine peut être adjointe à une formulation à base de Paracétamol selon l'invention, à différents dosages allant par exemple de 0,10 à 25mg de Phéniramine.

### - de la Méclozine :

La Méclozine peut être adjointe à une formulation à base de Paracétamol selon l'invention, à différents dosages aillant par exemple de 0,10 à 25mg de Méclozine.

### - de la Diphénhydremine :

La Diphénydramine peut être adjointe à une formulation à base de Paracétamol selon l'invention, à différents dosages allant par exemple de 2 à 25mg de Diphénydramine.

### - du Dimenhydrinate :

Le Dimenhydrinate peut être adjoint à une formulation à base de Paracétamol selon l'invention, à différents dosages allant par exemple de 2 à 25mg de Dimenhydrinate.

### - de la Ciproheptadine :

La Ciproheptadine peut être adjointe à une formulation à base de Paracétamol selon l'invention, à différents dosages allant par exemple de 0,10 à 2,0mg de Ciproheptadine.

La forme galénique selon l'invention peut également contenir en association avec du Paracétamol d'autres molécules actives usuellement utilisées en association avec le Paracétamol et pour lesquelles la formulation sous forme hydroalcoolique est pertinente. A titre d'exemple, on peut citer des formulations selon l'invention associant à du Paracétamol :

### - du Dextropropoxyfène :

Le Dextropropoxyfène peut être adjoint à une formulation à base de Paracétamol selon l'invention, à différents dosages allant par exemple de 5,0 à 15,0mg de Dextropropoxyfène.

### - de la Codéine :

La Codéine peut être adjointe à une formulation à base de Paracétamol selon l'invention, à différents dosages allant par exemple de 5,0 à 15,0mg de Codéine.

Bien entendu, l'invention n'est évidemment pas limitée aux exemples représentés et décrits ci-dessus, mais couvre au contraire toutes les variantes.

## Revendications

1. Forme galénique pour l'administration par voie trans-muqueuse d'au moins un principe actif, **caractérisée en ce que** ledit principe actif est du Paracétamol en état de dissolution stable et complète dans une solution hydroalcoolique comprenant entre 10 et 70% d'éthanol et entre 30 et 90% d'eau en masse, de façon à permettre une absorption rapide dudit principe actif à travers les muqueuses de la cavité buccale et/ou de l'oropharynx.

2. Forme galénique selon la revendication 1, **caractérisée en ce que** le degré d'alcool de la solution hydroalcoolique est compris entre 10° et 70°.

3. Forme galénique selon l'une des précédentes revendications, **caractérisée en ce que** le degré d'alcool de la solution hydroalcoolique est compris entre 25° et 55°.

4. Forme galénique selon l'une des précédentes revendications, **caractérisée en ce que** la solution hydroalcoolique comprend un ou plusieurs adjuvants de dissolution.

5. Forme galénique selon la revendication 4, **caractérisée en ce que** les adjuvants de dissolution sont choisis parmi les polymères de type Poly-éthylène glycol de faible poids moléculaire, les tensioactifs, l'alcool isopropylique et/ou des mélanges alcool-huile.

6. Forme galénique selon l'une des précédentes revendications, **caractérisée en ce que** la solution hydroalcoolique comprend un arôme et/ou un édulcorant pour adoucir la sensation gustative.

7. Forme galénique selon l'une des précédentes revendications, **caractérisée en ce que** la solution hydroalcoolique comprend un agent correcteur de pH.

8. Forme galénique selon l'une quelconque des revendications précédentes, **caractérisée en ce que** le volume d'administration est inférieur à 5 ml.

9. Forme galénique selon l'une quelconque des précédentes revendications, **caractérisée en ce qu'**elle comprend entre 25 et 250mg de Paracétamol pour des volumes de solution hydroalcoolique compris entre 0,5ml et 2,5ml.

10. Forme galénique selon l'une des précédentes revendications, **caractérisée en ce qu'**elle est conditionnée au moyen d'un contenant de petite taille souple ou rigide, garantissant la stabilité du ou des principe(s) actif(s) dans leur dissolution dans le temps, préférentiellement sous forme d'un étui étanche unidose ou multidoses d'une contenance maximum de 30ml.

11. Forme galénique selon l'une quelconque des précédentes revendications, **caractérisée en ce que** le Paracétamol est associé à au moins un autre principe actif thérapeutique.

12. Forme galénique selon la revendication 11, **caractérisée en ce que** l'autre principe actif thérapeutique est choisi parmi la PseudoEphédrine, la Triprolidine, la Prométhazine, la Phéniramine, la Méclozine, la Diphénhydramine, le Dimenhydrinate ou la Ciproheptadine.

13. Forme galénique selon la revendication 11, **caractérisée en ce que** l'autre principe actif thérapeutique est choisi parmi le Dextropropoxylène ou la Codéine.

14. Procédé de fabrication d'une forme galénique selon l'une des revendications 1 à 10, comprenant les étapes suivantes
- introduire sous agitation les deux tiers du Paracétamol dans l'éthanol,
- agiter la préparation,
- introduire sous agitation l'eau purifiée,
- agiter,
- introduire le tiers restant de Paracétamol, et
- agiter jusqu'à dissolution complète de la totalité du Paracétamol.

15. Procédé de fabrication d'une forme galénique selon l'une des revendications 1 à 10, comprenant les étapes suivantes :
- introduire sous agitation les deux tiers du Paracétamol dans l'éthanol,
- agiter la préparation durant 10 à 60 minutes,
- introduire sous agitation l'eau purifiée,
- agiter durant 10 à 60 minutes,
- introduire le tiers restant de Paracétamol,
- agiter jusqu'à dissolution complète de la totalité du Paracétamol, et
- filtrer.

16. Utilisation de la forme galénique selon l'une des revendications 1 à 10, pour la réalisation d'un médicament destiné au traitement de la douleur, d'états fiévreux ou de la migraine.

17. Utilisation de la forme galénique selon l'une des revendications 1 à 13, pour la réalisation d'un médicament destiné au traitement des affections de type grippal avec encombrements rhino-pharyngés.

## Claims

1. A galenic form for the transmucous administration of at least one active principle, **characterised in that** said active principle is paracetamol in a state of stable and complete dissolution in a water/alcohol solution comprising between 10% and 70% ethanol and between 30% and 90% water by mass, so as to enable said active principle to be absorbed rapidly through the mucosa of the mouth and/or of the oropharynx.

2. A galenic form according to claim 1, **characterised in that** the alcohol degree of the water/alcohol solution is between 10° and 70°.

3. A galenic form according to one of the preceding claims, **characterised in that** the alcohol degree of the water/alcohol solution is between 25° and 55°.

4. A galenic form according to one of the preceding claims, **characterised in that** the water/alcohol solution comprises one or more dissolution adjuvants.

5. A galenic form according to claim 4, **characterised in that** the dissolution adjuvants are chosen from polymers of the polyethylene glycol type with a low molecular weight, surfactants, isopropyl alcohol and/or alcohol/oil mixtures.

6. A galenic form according to one of the preceding claims, **characterised in that** the water/alcohol solution comprises a flavouring and/or a sweetener for sweetening the gustatory sensation.

7. A galenic form according to one of the preceding claims, **characterised in that** the water/alcohol solution comprises a pH correcting agent.

8. A galenic form according to any one of the preceding claims, **characterised in that** the administration volume is below 5 ml.

9. A galenic form according to any one of the preceding claims, **characterised in that** it comprises between 25 and 250 mg of paracetamol for volumes of water/alcohol solution of between 0.5 ml and 2.5 ml.

10. A galenic form according to one of the preceding claims, **characterised in that** it is packaged by means of a small flexible or rigid container guaranteeing the stability of the active principle or principles in the dissolution thereof over time, preferentially in the form of a single-dose or multi-dose sealed case with a maximum content of 30 ml.

11. A galenic form according to any one of the preceding claims, **characterised in that** the paracetamol is associated with at least one other therapeutic active principle.

12. A galenic form according to claim 11, **characterised in that** the other therapeutic active principle is chosen from pseudoephedrine, triprolidine, promethazine, pheniramine, meclozine, diphenhydramine, dimenhydrinate or cyproheptadine.

13. A galenic form according to claim 11, **characterised in that** the other therapeutic active principle is chosen from dextropropoxyfene or codeine.

14. A method of manufacturing a galenic form according to one of claims 1 to 10, comprising the following steps:
- introducing two thirds of the paracetamol under agitation into the ethanol,
- agitating the preparation,
- introducing the purified water under agitation,
- agitating,
- introducing the remaining third of the paracetamol, and
- agitating until all the paracetamol is completely dissolved.

15. A method of manufacturing a galenic form according to one of claims 1 to 10, comprising the following steps:
- introducing two thirds of the paracetamol under agitation into the ethanol,
- stirring the preparation for 10 to 60 minutes,
- introducing purified water under agitation,
- agitating for 10 to 60 minutes,
- introducing the remaining third of the paracetamol,
- agitating until all the paracetamol is completely dissolved,
and
- filtering.

16. Use of the galenic form according to one of claims 1 to 10 for producing a medication intended for the treatment of pain, feverish states or migraine.

17. Use of the galenic form according to one of claims 1 to 13 for producing a medication intended for the treatment of ailments of the influenza type with rhinopharyngal congestion.

## Patentansprüche

1. Galenische Form zur transmukosalen Verabreichung wenigstens eines Wirkstoffs, **dadurch gekennzeichnet, dass** der Wirkstoff Paracetamol im Zustand stabiler und vollständiger Auflösung in einer hydroalkoholischen Lösung mit 10 bis 70 Massenprozent Ethanol und 30 bis 90 Massenprozent Wasser ist, um eine schnelle Aufnahme des Wirkstoffs durch die Schleimhäute der Mundhöhle und/oder des Mundrachenraums zu ermöglichen.

2. Galenische Form nach Anspruch 1, **dadurch gekennzeichnet, dass** der Alkoholgehalt der hydroalkoholischen Lösung zwischen 10° und 70° liegt.

3. Galenische Form nach einem der vorhergehenden Ansprüche, **dadurch gekennzeichnet, dass** der Alkoholgehalt der hydroalkoholischen Lösung zwischen 25° und 55° liegt.

4. Galenische Form nach einem der vorhergehenden Ansprüche, **dadurch gekennzeichnet, dass** die hydroalkoholische Lösung ein oder mehrere Lösungshilfsmittel umfasst.

5. Galenische Form nach Anspruch 4, **dadurch gekennzeichnet, dass** die Lösungshilfsmittel unter den Polymeren vom Typ Polyethylenglykol mit geringem Molekulargewicht, den oberflächenaktiven Stoffen, Isopropylalkohol und/oder Alkohol/Öl-Mischungen ausgewählt sind.

6. Galenische Form nach einem der vorhergehenden Ansprüche, **dadurch gekennzeichnet, dass** die hydroalkoholische Lösung ein Aroma und/oder ein Süßungsmittel umfasst, um das Geschmacksempfinden angenehmer zu gestalten.

7. Galenische Form nach einem der vorhergehenden Ansprüche, **dadurch gekennzeichnet, dass** die hydroalkoholische Lösung ein pH-Korrekturmittel umfasst.

8. Galenische Form nach einem der vorhergehenden Ansprüche, **dadurch gekennzeichnet, dass** das Verabreichungsvolumen weniger als 5 ml beträgt.

9. Galenische Form nach einem der vorhergehenden Ansprüche, **dadurch gekennzeichnet, dass** sie zwischen 25 und 250 mg Paracetamol für Volumina der hydroalkoholischen Lösung zwischen 0,5 ml und 2,5 ml enthält.

10. Galenische Form nach einem der vorhergehenden Ansprüche, **dadurch gekennzeichnet, dass** sie verpackt ist mittels eines weichen oder starren Behälters kleiner Größe, der die Stabilität des Wirkstoffs oder der Wirkstoffe in ihrer Auflösung über die Zeit sicherstellt, vorzugsweise in Form einer dichten Hülle für eine Dosis oder mehrere Dosen mit einem maximalen Fassungsvermögen von 30 ml.

11. Galenische Form nach einem der vorhergehenden Ansprüche, **dadurch gekennzeichnet, dass** das Paracetamol mit wenigstens einem weiteren therapeutischen Wirkstoff kombiniert ist.

12. Galenische Form nach Anspruch 11, **dadurch gekennzeichnet, dass** der weitere therapeutische Wirkstoff aus Pseudoephedrin, Triprolidin, Promethazin, Pheniramin, Meclozin, Diphenhydramin, Dimenhydrinat oder Cyproheptadin ausgewählt ist.

13. Galenische Form nach Anspruch 11, **dadurch gekennzeichnet, dass** der weitere therapeutische Wirkstoff aus Dextropropoxyphen oder Codein ausgewählt ist.

14. Verfahren zur Herstellung einer galenischen Form nach einem der Ansprüche 1 bis 10, welches die folgenden Schritte umfasst:
- Einbringen von zwei Dritteln Paracetamol unter Rühren in das Ethanol,
- Rühren der Zubereitung,
- Einbringen des gereinigten Wassers unter Rühren,
- Rühren,
- Einbringen des verbleibenden Drittels Paracetamol und
- Rühren bis zur vollständigen Auflösung des gesamten Paracetamol.

15. Verfahren zur Herstellung einer galenischen Form nach einem der Ansprüche 1 bis 10, das die folgenden Schritte umfasst:
- Einbringen von zwei Dritteln Paracetamol unter Rühren in das Ethanol,
- Rühren der Zubereitung für 10 bis 60 Minuten,
- Einbringen des gereinigten Wassers unter Rühren,
- Rühren für 10 bis 60 Minuten,
- Einbringen des verbleibenden Drittels Paracetamol,
- Rühren bis zur vollständigen Auflösung des gesamten Paracetamol und
- Filtrieren.

16. Verwendung der galenischen Form nach einem der Ansprüche 1 bis 10, für die Herstellung eines Medikaments zur Behandlung von Schmerzen, Fieberzuständen oder Migräne.

17. Verwendung der galenischen Form nach einem der Ansprüche 1 bis 13, für die Herstellung eines Medikaments zur Behandlung von Erkrankungen grippaler Art mit Verstopfung von Nase und Rachenraum.
